# EUROPEAN PATENT SPECIFICATION

(11) **EP 4 289 419 B1**
(45) Date of publication and mention of the grant of the patent: **29.01.2025**
(21) Application number: 23177238.5
(22) Date of filing: 05.06.2023
(51) Int. Cl.: A61K 31/65, A61K 9/00, A61K 47/02, A61K 47/18, A61K 9/19

(54) **A FREEZE DRIED PARENTERAL COMPOSITION OF OMADACYCLINE TOSYLATE WITH IMPROVED STABILITY**
GEFRIERGETROCKNETE PARENTERALE ZUSAMMENSETZUNG VON OMADACYCLIN TOSYLAT MIT VERBESSERTER STABILITÄT
COMPOSITION PARENTÉRALE LYOPHILISÉE DE TOSYLATE D'OMADACYCLINE PRÉSENTANT UNE STABILITÉ AMÉLIORÉE

(30) Priority: 11.06.2022 IN 202223033552
(43) Date of publication of application: 13.12.2023
(73) Proprietor: Gufic Biosciences Limited, Kabilpore, Gujarat 396424 (IN)
(72) Inventor: PATEL, Mitesh Natavarlal, 396 424 Navsari, Gujarat (IN); DAVE, Mafatlal Tribhovandas, 396 424 Navsari, Gujarat (IN); ANKLESARIA, Beena Pourusashap, 396 424 Navsari, Gujarat (IN); CHOKSI, Pranavkumar Jayesh, 396 424 Navsari, Gujarat (IN)
(74) Representative: Isarpatent

(56) References cited:
- WO-A1-2018/026987
- CA-A1- 3 179 596
- IZUTSU K I ET AL: "Effect of counterions on the physical properties of l-arginine in frozen solutions and freeze-dried solids", INTERNATIONAL JOURNAL OF PHARMACEUTICS, ELSEVIER, NL, vol. 301, no. 1-2, 14 September 2005 (2005-09-14), pages 161 - 169, XP027624254, ISSN: 0378-5173, [retrieved on 20050914]

## Description

### Technical Field of the Invention:

The present invention relates to a freeze dried pharmaceutical composition for parenteral administration, with improved stability and reduced impurities, comprising Omadacycline Tosylate with sodium bisulfite and arginine as stabilizing agents. The invention further relates to a process for preparation thereof.

### Background of the Invention:

In the past 15 to 20 years the threat of bioterrorism has increased as a result of increasing political and economic unrest in many parts of the world. The Centres for Disease Control (CDC) has classified bioterrorism agents into three categories based on their potential to cause severe disease that results in high rates of mortality, and according to how readily these agents can be disseminated in the general population. Among the bioterrorism agents that pose the highest threat are *Bacillus anthracis* and *Yersinia pestis,* which are the causative pathogens for anthrax and plague, respectively. Current antibiotic treatment options against these Category A Bio threat pathogens are limited and the potential for engineered antibiotic resistance is high, thus, new therapeutic options are needed for prophylaxis and treatment of the diseases caused by these pathogens. Few new oral antibiotics are in development for the treatment of bio threat pathogens, and those older agents that have been approved are facing increasing resistance problems and could face engineered resistance.

Omadacycline is a novel aminomethylcycline of the tetracycline family, designed to overcome mechanisms of resistance to the tetracycline class .The extensive preclinical and clinical development program for Omadacycline is based on its demonstrated potent activity against key pathogens for serious community-acquired infections, including methicillin-resistant *Staphylococcus aureus,* multidrug resistant *Streptococcus pneumoniae,* Gram-negative aerobes, and atypical pathogens, and its lack of cross resistance to older generation tetracyclines and other antibiotic classes.

Omadacycline is currently in clinical development for acute bacterial skin and skin structure infection (ABSSSI) and community acquired bacterial pneumonia (CABP) as oral and intravenous (IV) monotherapy. Because of its broad *in vitro* spectrum of activity, clinical profile, and oral bioavailability, Omadacycline could be well suited for use in the treatment or post-exposure prophylaxis of infections of concern in both the biodefense and public health settings. This study evaluated the *in vitro* and *invivo* activity of Omadacycline against *B. anthracis* and *Y. pestis.*

Omadacycline tosylate, an aminomethylcycline is a semisynthetic derivative of the tetracycline class of antibacterial drugs, for intravenous or oral administration. The chemical name of Omadacycline tosylate is (4S,4aS,5aR,12aS)-4,7-bis(dimethylamino)-9-(2,2-dimethylpropylaminomethyl)-3,10,12,12a-tetrahydroxy-1,11-dioxo-1,4,4a,5,5a,6,11,12a-octa hydrotetracene-2-carboxamide, 4-methylbenzenesulfonate. The molecular formula is C36H48N4O10S (monotosylate salt) and the molecular weight is 728.9 (monotosylate salt). The following represents the chemical structure of omadacycline tosylate:

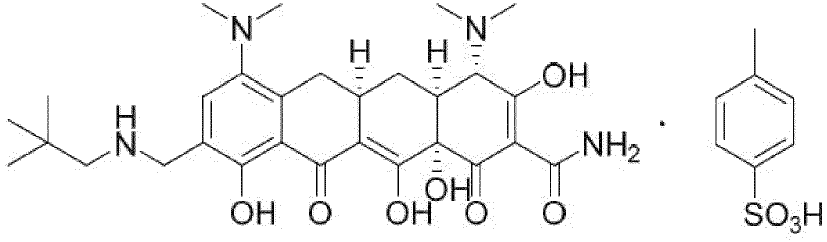

Omadacycline is marketed as lyophilized product for intravenous infusion by Paratek Pharmaceuticals, Inc.Boston, MA, USA under the trade name NUZYRA^{®}. Omadacycline is indicated for the treatment of adult patients for complicated skin and skin structure infections, complicated intra-abdominal infections and community-acquired bacterial pneumonia.

Omadacycline Tosylate is generally unstable to heat, humidity, acid and alkali, and forms degradation product, epimer, hence, it is necessary to develop a pharmaceutical composition which stabilizes the active compound and salt thereof for parenteral administration.

Currently available formulation of Omadacycline is in lyophilized vial containing sucrose as Lyoprotectant which does not provide desirable stability due to the sensitivity of Omadacycline against environmental stress. NUZYRA for Injection needs to be stored at 20°C to 25°C (68°F to 77°F). This Product is unstable while storage at 40°C, 75% RH(Accelerated condition) considering Zone IV-B for Indian market, Impurity profile for Total impurities rise up to around 6.0% at 40°C, 75% RH after 3 months. Once the vial is reconstituted and diluted for infusion, it should be used within 1 hour. Therefore, there is a need for stable pharmaceutical composition comprising of Omadacycline. Moreover, it would be desirable to provide compositions having a good stability and a long shelf life.

The Applicant's earlier filed Indian Patent Application no. 202121018095 discloses a freeze dried pharmaceutical composition for parenteral administration comprising arginine as a stabilizing agent.

The present inventors found that there is a scope to provide a pharmaceutical composition of Omadacycline tosylate for parenteral administration with further improved stability, reduced impurities thereby increasing the shelf life using stabilizing agent sodium bisulfite in combination with arginine. This remains the objective of the present invention.

### Summary of the Invention:

In accordance with above, the present invention provides a freeze dried pharmaceutical composition of Omadacycline Tosylate for parenteral administration with improved stability and reduced impurities, using sodium bisulfite together with arginine as stabilizing agents.

In an aspect, the present invention provides a freeze dried pharmaceutical composition for parenteral administration comprising;
i. Omadacycline Tosylate equivalent to Omadacycline in an amount ranging between 50mg to 100mg/vial,
ii. Arginine in an amount ranging between 50mg to 200 mg/ vial in combination with sodium bisulfite in an amount ranging between 0.5 mg to 2 mg/ vial as stabilizing agents,
iii. Dilute sodium hydroxide solution q.s, and/or
iv. Dilute hydrochloric acid solution q.sto regulate pH
wherein pH of the bulk solution is in the range of 5.0 to 6.6 before freeze drying.

In another aspect, the pharmaceutical composition of Omadacycline Tosylate and stabilizing agents are freeze dried and provided as a drug concentrate.

In yet another aspect, the present invention provides a process for increasing the stability and reducing the impurities of freeze-dried pharmaceutical composition of Omadacycline tosylate in an aqueous solution comprising a step of combining Omadacycline Tosylate with Arginine and sodium bisulfite as stabilizing agents in aqueous medium and maintaining the bulk solution within pH range of 5.6 to 6.6.

### Detailed description of the Invention:

The invention will now be described in detail in connection with certain preferred and optional embodiments, so that various aspects thereof may be fully understood and appreciated.

The term 'composition/formulation' referred interchangeably in the entire description refers and means to the freeze dried pharmaceutical composition of Omadacycline Tosylate for parenteral administration.

In an embodiment, the present invention relates to a freeze dried pharmaceutical composition for parenteral administration comprising;
i. Omadacycline Tosylate equivalent to Omadacycline,
ii. Arginine in combination with sodium bisulfite as stabilizing agents,
iii. Dilute sodium hydroxide solution, and/or
iv. Dilute hydrochloric acid solution to regulate pH
wherein the pH of the bulk solution is in the range of 5.0 to 6.6before freeze drying.

In an embodiment, Omadacycline Tosylateequivalent to Omadacycline as active ingredient is present in an amount of 50-100mg/vial.

In another embodiment, Arginineas stabilizing agent in the present composition is in an amount ranging between 50mg to 200 mg/ vial, morepreferably, between 100mg to 200mg/ vialand sodium bisulfite is in an amount ranging between0.5 mg to 2mg/ vial, preferably between 0.5mg to 1.0mg/vial.

In an embodiment, Omadacycline Tosylate equivalent to Omadacycline and Arginine is present in the ratio of 1:1 to 1:2, preferably in the ratio 1:1 and Omadacycline Tosylate equivalent to Omadacycline and Sodium bisulfite is present in the ratio of 1: 0.005 to 1: 0.02, preferably in the ratio 1:0.01.

The pH of the composition is maintained within the range of 5.0to 6.6 using dilute sodium hydroxide solution and/or dilute hydrochloric acid solution in q.s.

In an embodiment, the pre-lyophilized formulation of Omadacycline tosylate and sodium bisulfite along with arginine as stabilizing agents is lyophilized and provided as a drug concentrate, suitable for parenteral administration.

Accordingly, the present invention discloses a freeze dried pharmaceutical composition for parenteral administration comprising;
a) Omadacycline tosylate equivalent to Omadacycline in an amount of 50mg to 100mg/vial;
b) Arginine in an amount of 50mg to 200mg/vial;
c) Sodium bisulfite in an amount of 0.5 mg to 2 mg/ vial;
d) dilute sodium hydroxide solution q.s. and/ or
e) dilute hydrochloric acid solution q.s.to regulate pH;
wherein pH of the bulk solution is in the range of 5.0 to 6.6 before freeze drying.

In a preferred embodiment, the present invention discloses a freeze dried pharmaceutical composition for parenteral administration comprising;
a) Omadacycline tosylate equivalent to Omadacycline in an amount of 100mg/vial;
b) Arginine in an amount of 100mg/vial;
c) Sodium bisulfite in an amount of 1 mg/ vial;
d) dilute sodium hydroxide solution q.s. and/or
e) dilute hydrochloric acid solution q.s.to regulate pH;
wherein pH of the bulk solution is in the range of 5.0 to 6.6 before freeze drying.

In another embodiment, the present invention discloses a process for increasing the stability and reducing the impurities of freeze-dried pharmaceutical composition of Omadacycline tosylate in an aqueous solution comprising a step of combining Omadacycline Tosylate with Arginine and sodium bisulfite as stabilizing agents in aqueous medium and maintaining the bulk solution within pH range of 5.6 to 6.6.

In yet another embodiment, the lyophilized formulation of Omadacyline tosylate of the present invention is subjected to accelerated degradation studies at 40°C with relative humidity of 75% moisture for six months and compared with the Applicant's earlier filed Omadacyline tosylate composition and presented as trial 5 below, wherein the Omadacyline tosylate composition exhibits improved stability and reduced impurities over the earlier formulation.

In another embodiment, the invention provides a process for the manufacture of a stable freeze dried pharmaceutical composition of Omadacycline Tosylate comprising the following steps:
a) Preparing an aqueous solution of arginine in water for injection;
b) Adjusting the pH of the solution prepared in step (a) to between 3.8 to 4.5 with dilute hydrochloric acid solution;
c) Adding and dissolving sodium bisulfite in solution step (b), cooling the solution between temperature of 2°C to 8°C and maintaining the temperature throughout the manufacturing under nitrogen purging;
d) Dissolving Omadacycline Tosylate in the solution obtained in step (c);
e) Adjusting the pH of the Solution prepared in step (d) to between 5.6 to 6.6 with Dilute Hydrochloric acid solution or Dilute sodium hydroxide solution and making up the volume to batch size with water for injection;
f) Filtering the solution obtained in step (e) and filling the vials;
g) Freezing the solution filled in vials obtained in step (f); and
h) Freeze drying the frozen solution filled in vials obtained in step (g).

The process of freeze drying comprises the following steps:
a) freezing the solutionof Omadacycline Tosylate filled in vials at a temperature below -35°C and maintaining said temperature for at least 3 hours;
b) primary drying of the frozen solution of step (a) under vacuum from 50 mtorr to 100mtorr and at a temperature between -30 and 15°C, and maintaining said conditions for at least 50 hours; and
c) secondary drying of the primary dried frozen Solution of step (b) under vacuum from 10mtorr to 50mtorr and at a temperature between 15°C and 40°C, and maintaining said conditions for at least 8 hours, to achieve water content not more than 2.0%w/w.

In an embodiment, the composition of the present composition exhibits good stability with less than 5% impurities even after 6 months at 40°C (Accelerated studies) with relative humidity of 75% moisture and in lyophilized form.

The composition of the present invention after freeze drying is chemically and physically stable over an extended period of time and is suitable for intended pharmaceutical use after reconstitution with water for injection, 0.9% w/v sodium chloride injection or 5% w/v dextrose injection.

The freeze dried Omadacycline tosylate composition provided in accordance with this invention when reconstituted with 5 ml of suitable vehicle contains final drug concentrate of 20 mg/ml and subsequently diluted with 5% Dextrose injection, to a final concentration of 1mg/ml to 2mg/ml.

The freeze dried drug may be diluted with suitable diluents before administration as IV injection. The final concentration of solution may be reduced to further desired level using 5% Dextrose infusion prior to administration to a patient.

The pharmaceutical composition of the present invention is administered to a patient according to the dosing regimen. It should be understood that the specific dosing regimen for any particular patient will depend on the age, body weight, general health, diet, sex, specific decease treated and severity of the condition and such variable factors and judgement of the physician.

The pharmaceutical composition of the present invention is useful in the treatment of various gram-positive and gram-negative bacterial infections such as complicated skin and skin structure infections, complicated intra-abdominal infections and community-acquired bacterial pneumonia.

### Industrial Advantages:

1. The pharmaceutical composition comprising Omadacycline Tosylate as active with sodium bisufite together with Arginine as stabilizing agentshas less than 5% impurities even after 6 months at 40°C (accelerated studies) in lyophilized form.

Other features and embodiments of the invention will become apparent by the following examples which are given for illustration of the invention rather than limiting its intended scope. Various changes and modifications to the disclosed embodiments will be apparent to those skilled in the art.

### Experimental:

### Example 1: Composition (Earlier Application)

| **Ingredients** | | **Qty/vial** |
|---|---|---|
| Omadacycline eq.to.Omadacycline | Tosylate | 100mg |
| Arginine | | 100mg |
| pH of bulk solution adjusted with Dilute HCL solution/Dilute NaOH solution | | 4.35 |

### Trial 1:

Dissolved 2.0 gm of Arginine in 42ml Water for injection and added3.5 ml of 3M hydrochloric acid solution to adjust pH to 7.8 to 8.0 and cooled the solution between temperature of 2°C to 8°C and maintained the temperature throughout the manufacturing under nitrogen purging. 2.620 gm of Omadacycline Tosylate equivalent to 2.0 gm Omadacycline free base was then added. After the Active was added, the pH was adjusted to 4.0 to 4.5 using 0.1 M hydrochloric acid solution, as appropriate. The volume of the resulting solution was adjusted to 60ml with additional water for injection. The mixture was then filtered through a sterile 0.22 pm filter. Type 1 glass vials were then filled with 3.0 ml of the solution per vial. The solution in each vial was lyophilized using freeze dryer as per following method,
The process of freeze drying comprises the following steps:
a) freezing the solution at a temperature below -35°C and maintaining said temperature for at least 3 hour;
b) primary drying the frozen solution of step (a) under vacuum from 50 mtorr to 100 mtorr and at a temperature between -30 and 15°C, and maintaining said conditions for at least 50 hours; and
c) secondary drying the primary dried frozen Solution of step (b) under vacuum from 10 mtorr to 50 mtorr and at a temperature between 15°C and 40°C, and maintaining said conditions for at least 8 hours, thereby obtaining the lyophilized compositions each comprising 100 mg of Omadacycline.

The resulting lyophilized preparation was stored at different storage temperatures at different time intervals and the residue of Omadacycline was tested.

| Period | | Assay (%) | pH | Total impurities (%) | Storage temp. | Colour |
|---|---|---|---|---|---|---|
| Initial | Bulk solution | 99.28 | 4.19 | 0.68 | 2-8° C | Clear light orange colour solution |
| | Lyophilized | 99.21 | *4.09 | 0.72 | 25° C | Orange colour lyophilized cake |
| After 3 months | | 96.75 | *4.12 | 3.15 | 40°C | Dark Orange colour lyophilized cake |

| | | | | | | |
|---|---|---|---|---|---|---|
| *for determination of pH - one lyophilized vial reconstituted with 5 ml water for Injection. | | | | | | |

As is evident from the above trial 1, when the pH of the bulk solution is adjusted between 4.0 to 4.5; the composition was unstable and exhibits more impurities after 3months.

### Trial 2:

Dissolved 2.0 gm of Arginine in 42ml Water for injection and added3.5 ml of 3M hydrochloric acid solution were also added to adjust pH to 7.8 to 8.0 and cooled the solution between temperature of 2°C to 8°C and maintained the temperature throughout the manufacturing under nitrogen purging. 2.620 gm of Omadacycline Tosylate equivalent to 2.0 gm Omadacycline free base was then added. After the Active was added, the pH was adjusted to 4.6 to 5.5 using 0.1 M hydrochloric acid solution, as appropriate. The volume of the resulting solution was adjusted to 60ml with additional water for injection. The mixture was then filtered through a sterile 0.22 pm filter. Type 1 glass vials were then filled with 3.0 ml of the solution per vial. The solution in each vial was lyophilized using freeze dryer as per following method,
The process of freeze drying comprises the following steps:
a) Freezing the solution at a temperature below -35°C and maintaining said temperature for at least 3 hour;
b) primary drying the frozen solution of step (a) under vacuum from 50mtorr to 100m torr and at a temperature between -30 and 15°C, and maintaining said conditions for at least 50 hours; and
c) secondary drying the primary dried frozen Solution of step (b) under vacuum from 10 mtorr to 50 mtorr and at a temperature between 15°C and 40°C, and maintaining said conditions for at least 8 hours, thereby obtaining the lyophilized compositions each comprising 100 mg of Omadacycline.

The resulting lyophilized preparation was stored at different storage temperatures at different time intervals and the residue of Omadacycline was tested.

| Period | | Assay (%) | pH | Total impurities (%) | Storage temp. | Colour |
|---|---|---|---|---|---|---|
| Initial | Bulk solution | 99.24 | 5.09 | 0.69 | 2-8° C | Clear light orange colour solution |
| | Lyophilized | 99.19 | *4.88 | 0.74 | 25° C | Orange colour lyophilized cake |
| After 3 months | | 97.95 | *4.83 | 1.95 | 40°C | Orange colour lyophilized cake |

| | | | | | | |
|---|---|---|---|---|---|---|
| *for determination of pH - one lyophilized vial reconstituted with 5 ml water for Injection. | | | | | | |

As is evident from the above trial 2, when the pH of the bulk solution is adjusted between 4.6 to 5.5; the composition was also unstable and exhibits more impurities after 3months.

### Trial 3:

Dissolved 2.0 gm of Arginine in 42ml Water for injection and added3.5 ml of 3M hydrochloric acid solution were also added to adjust pH to 7.8 to 8.0 and cooled the solution between temperature of 2°C to 8°C and maintained the temperature throughout the manufacturing under nitrogen purging., 2.620 gm of Omadacycline Tosylate equivalent to 2.0 gm Omadacycline free base was then added. After the Active was added, the pH was adjusted to 5.6 to 6.6 using 0.1 M hydrochloric acid solution, as appropriate. The volume of the resulting solution was adjusted to 60ml with additional water for injection. The mixture was then filtered through a sterile 0.22 pm filter. Type 1 glass vials were then filled with 3.0 ml of the solution per vial. The solution in each vial was lyophilized using freeze dryer as per following method,
The process of freeze drying comprises the following steps:
a) Freezing the solution at a temperature below -35°C and maintaining said temperature for at least 3 hour;
b) primary drying the frozen solution of step (a) under vacuum from 50 mtorr to 100 mtorr and at a temperature between -30 and 15°C, and maintaining said conditions for at least 50 hours; and
c) secondary drying the primary dried frozen Solution of step (b) under vacuum from 10 mtorr to 50 mtorr and at a temperature between 15°C and 40°C, and maintaining said conditions for at least 8 hours, thereby obtaining the lyophilized compositions each comprising 100 mg of Omadacycline.

The resulting lyophilized preparation was stored at different storage temperatures at different time intervals and the residue of Omadacycline was tested.

| Period | | Assay (%) | pH | Total impurities (%) | Storage temp. | Colour |
|---|---|---|---|---|---|---|
| Initial | Bulk solution | 99.34 | 6.30 | 0.62 | 2-8° C | Clear light orange colour solution |
| | Lyophilized | 99.29 | *5.91 | 0.68 | 25° C | Orange colour lyophilized cake |
| After 3 months | | 98.51 | *5.85 | 1.45 | 40°C | Orange colour lyophilized cake |
| After 6 months | | 94.71 | *5.87 | 5.26 | 40°C | Orange colour lyophilized cake |

| | | | | | | |
|---|---|---|---|---|---|---|
| *for determination of pH - one lyophilized vial reconstituted with 5 ml water for Injection. | | | | | | |

### Trial 4:

Dissolved 4.0 gm of Arginine in 38ml Water for injection and added3.8 ml of 3M hydrochloric acid solution were also added to adjust pH to 7.8 to 8.0 and cooled the solution between temperature of 2°C to 8°C and maintained the temperature throughout the manufacturing under nitrogen purging, 2.620 gm of Omadacycline Tosylate equivalent to 2.0 gm Omadacycline free base was then added. After the Active was added, the pH was adjusted to 5.6 to 6.6 using 0.1 M hydrochloric acid solution, as appropriate. The volume of the resulting solution was adjusted to 60ml with additional water for injection. The mixture was then filtered through a sterile 0.22 pm filter. Type 1 glass vials were then filled with 3.0 ml of the solution per vial. The solution in each vial was lyophilized using freeze dryer as per following method,
The process of freeze drying comprises the following steps:
a) Freezing the solution at a temperature below -35°C and maintaining said temperature for at least 3 hour;
b) primary drying the frozen solution of step (a) under vacuum from 50 mtorr to 100 mtorr and at a temperature between -30 and 15°C, and maintaining said conditions for at least 50 hours; and
c) secondary drying the primary dried frozen Solution of step (b) under vacuum from 10 mtorr to 50 mtorr and at a temperature between 15°C and 40°C, and maintaining said conditions for at least 8 hours, thereby obtaining the lyophilized compositions each comprising 100 mg of Omadacycline.

The resulting lyophilized preparation was stored at different storage temperatures at different time intervals and the residue of Omadacycline was tested.

| Period | | Assay (%) | pH | Total impurities (%) | Storage temp. | Colour |
|---|---|---|---|---|---|---|
| Initial | Bulk solution | 99.33 | 6.34 | 0.61 | 2-8° C | Clear light orange colour solution |
| | Lyophilized | 99.26 | *5.92 | 0.70 | 25° C | Orange colour yophilized cake |
| After 3 months | | 98.44 | *5.84 | 1.48 | 40°C | Orange colour lyophilized cake |
| After 6 months | | 94.67 | *5.86 | 5.28 | 40°C | Orange colour lyophilized cake |

| | | | | | | |
|---|---|---|---|---|---|---|
| *for determination of pH - one lyophilized vial reconstituted with 5 ml water for Injection. | | | | | | |

It is observed that the total impurities increased more than 5% in trial number 3 and 4 when formulation was stored at 40°C in lyophilized form after 6 months.

In order to improve the stability and to control total impurities at 40°C, new trial has been conducted using Sodium bisulfite as stabilizing agent with Arginine.

### Example 2: Present Composition:

| **Sr. No.** | **Ingredients** | **Qty./vial** |
|---|---|---|
| 01 | Omadacycline tosylate equivalent to Omadacycline | 100 mg |
| 02 | Sodium Bisulfite | 1 mg |
| 03 | Arginine base | 100 mg |
| 04 | Dilute Sodium hydroxide solution for pH adjustment | q.s. |
| 05 | Dilute Hydrochloric acid Solution for pH adjustment | q.s. |
| 06 | Water for injection q.s. to | 3 ml |
| pH limit : 5.0 to 6.5 (bulk solution stage) | | |

### Process:

The general process for manufacture of freeze dried pharmaceutical composition of Omadacycline Tosylate is as follows:
a) Preparing an aqueous solution of arginine in water for injection;
b) Adjusting the pH of the solution prepared in step (a) to between 3.8 to 4.5 with dilute hydrochloric acid solution;
c) Adding and dissolving sodium bisulfite in solution step (b), cooling the solution between temperature of 2°C to 8°C and maintaining the temperature throughout the process under nitrogen purging;
d) Dissolving Omadacycline Tosylate in the solution obtained in step (c);
e) Adjusting the pH of the Solution prepared in step (d) to between 5.6 to 6.6 with Dilute Hydrochloric acid solution or dilute sodium hydroxide solution and making up the volume to batch size with water for injection;
f) Filtering the solution obtained in step (e) and filling the vials;
g) Freezing the Solution filled in vials obtained in step (f); and
h) Freeze drying the frozen solution filled in vials obtained in step (g).

### Trial 5:

Dissolved 2.0 gm of Arginine in 42 ml Water for injection and 3M hydrochloric acid solution was added to adjust pH between3.8 to 4.5. This was followed by addition and dissolution of 20 mg Sodium bisulfite to the above solution. Cooled the solution between temperature of 2°C to 8°C and maintained the temperature throughout the manufacturing under nitrogen purging, 2.620 gm of Omadacycline Tosylate (as active) equivalent to 2.0 gm Omadacycline free base was then added. After the Active was added, the pHs was adjusted to 5.6 to 6.6 using 0.1M hydrochloric acid solution or dilute sodium hydroxide solution, as appropriate. The volume of the resulting solution was adjusted to 60 ml with additional water for injection. The mixture was then filtered through a sterile 0.22 pm filter. Type 1 glass vials were then filled with 3.0 ml of the solution per vial. The solution in each vial was lyophilized using freeze dryer as per following method,
The process of freeze drying comprises the following steps:
a) Freezing the solution at a temperature below -35°C and maintaining said temperature for at least 3 hour;
b) primary drying the frozen solution of step (a) under vacuum from 50 mtorr to 100 mtorr and at a temperature between -30 and 15°C, and maintaining said conditions for at least 50 hours; and
c) secondary drying the primary dried frozen Solution of step (b) under vacuum from 10 mtorr to 50 mtorr and at a temperature between 15°C and 40°C, and maintaining said conditions for at least 8 hours, thereby obtaining the lyophilized compositions each comprising 100 mg of Omadacycline.

The resulting lyophilized preparation was stored at different storage temperatures at different time intervals and the residue of Omadacycline was tested.

| Period | | Assay (%) | pH | Total impurities (%) | Storage temp. | Colour |
|---|---|---|---|---|---|---|
| Initial | Bulk solution | 99.31 | 6.28 | 0.65 | 2-8° C | Clear light orange colour solution |
| | Lyophilized | 99.12 | *5.95 | 0.78 | 25° C | Orange colour lyophilized cake |
| After 3 months | | 98.21 | *5.89 | 1.75 | 40°C | Orange colour lyophilized cake |
| After 6 months | | 96.28 | *5.91 | 3.66 | 40°C | Orange colour lyophilized cake |

| | | | | | | |
|---|---|---|---|---|---|---|
| *for determination of pH - one lyophilized vial reconstituted with 5 ml water for Injection. | | | | | | |

It was observed that the composition comprising Omadacycline Tosylate equivalent to Omadacycline 100 mg and Arginine 100 mg and Sodium bisulfite 1 mg within pH range of 5.6 to 6.6 at bulk solution stage exhibits good stability with less than 5% impurities even after 6 months at 40°C in lyophilized form as shown in **Trial** 5.

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. A stable freeze-dried pharmaceutical composition for parenteral administration comprising,
a) Omadacycline Tosylate in an amount equivalent to 50 mg to 100mg Omadacycline/vial; and
b) Arginine in an amount of 50 mg to 200 mg/vial and Sodium bisulfite in an amount of 0.5 mg to 2 mg/vial as stabilizing agents;
c) dilute sodium hydroxide solution q.s. and/or
d) dilute hydrochloric acid solution q.s.to regulate pH;
wherein the pH of the solution, prior to lyophilisation, isin the range of 5.6-6.6.

2. The stable freeze-dried pharmaceutical composition as claimed in claim 1, wherein Omadacycline Tosylate equivalent to Omadacycline and Arginine is in the ratio of 1: 1 to 1:2 and Omadacycline Tosylate equivalent to Omadacycline and Sodium bisulfite is in the ratio of 1:0.005 to 1: 0.02.

3. The stable freeze-dried pharmaceutical composition as claimed in claim 1 and 2, comprising;
a) Omadacycline Tosylate in an amount of 100mg Omadacycline/vial;
b) Arginine in an amount of 100 mg/vial and Sodium bisulfite in an amount of 1 mg/vial as stabilizing agents;
c) dilute sodium hydroxide solution q.s. and/or
d) dilute hydrochloric acid solution q.s. to regulate pH;
wherein the pH of the solution, prior to lyophilisation, isin the range of 5.6-6.6.

4. The stable freeze dried pharmaceutical composition as claimed in any of the claims 1 to 3, wherein the process for increasing the stability and reducing the impurities of freeze-dried pharmaceutical composition of Omadacycline tosylate in an aqueous solution comprising a step of combining Omadacycline Tosylate with Arginine and sodium bisulfite as stabilizing agents in aqueous medium and maintaining the bulk solution within pH range of 5.6 to 6.6.

5. The stable freeze-dried pharmaceutical composition as claimed in claim 4,wherein the lyophilized form has less than 5% impurities even after 6 months at 40°C and 75%RH.

## Patentansprüche

1. Stabile gefriergetrocknete pharmazeutische Zusammensetzung zur parenteralen Verabreichung, umfassend,
a) Omadacyclin-Tosylat in einer Menge äquivalent zu 50 mg bis 100 mg Omadacyclin/Fläschchen; und
b) Arginin in einer Menge von 50 mg bis 200 mg/Fläschchen und Natriumbisulfit in einer Menge von 0,5 mg bis 2 mg/Fläschchen als Stabilisierungsmittel;
c) verdünnte Natriumhydroxidlösung q.s. und/oder
d) verdünnte Salzsäurelösung q.s. zur Regulierung des pH-Wertes;
wobei der pH-Wert der Lösung vor der Lyophilisierung im Bereich von 5,6-6,6 liegt.

2. Stabile gefriergetrocknete pharmazeutische Zusammensetzung, wie in Anspruch 1 beansprucht, wobei das Omadacyclin-Tosylat äquivalent zu Omadacyclin und Arginin im Verhältnis von 1:1 bis 1:2 vorliegt und das Omadacyclin-Tosylat äquivalent zu Omadacyclin und Natriumbisulfit im Verhältnis von 1:0,005 bis 1:0,02 vorliegt.

3. Stabile gefriergetrocknete pharmazeutische Zusammensetzung, wie in Anspruch 1 und 2 beansprucht, umfassend;
a) Omadacyclin-Tosylat in einer Menge von 100 mg Omadacyclin/Fläschchen;
b) Arginin in einer Menge von 100 mg/Fläschchen und Natriumbisulfit in einer Menge von 1 mg/Fläschchen als Stabilisierungsmittel;
c) verdünnte Natriumhydroxidlösung q.s. und/oder
d) verdünnte Salzsäurelösung q.s. zur Regulierung des pH-Wertes;
wobei der pH-Wert der Lösung vor der Lyophilisierung im Bereich von 5,6-6,6 liegt.

4. Stabile gefriergetrocknete pharmazeutische Zusammensetzung, wie in einem der Ansprüche 1 bis 3 beansprucht, wobei das Verfahren zur Erhöhung der Stabilität und zur Verringerung der Verunreinigungen der gefriergetrockneten pharmazeutischen Zusammensetzung von Omadacyclin-Tosylat in einer wässrigen Lösung einen Schritt eines Kombinierens von Omadacyclin-Tosylat mit Arginin und Natriumbisulfit als Stabilisierungsmittel in einem wässrigen Medium und eines Haltens der Hauptlösung im pH-Bereich von 5,6 bis 6,6 umfasst.

5. Stabile gefriergetrocknete pharmazeutische Zusammensetzung, wie in Anspruch 4 beansprucht, wobei die gefriergetrocknete Form selbst nach 6 Monaten bei 40°C und 75% RH weniger als 5% Verunreinigungen aufweist.

## Revendications

1. Composition pharmaceutique stable lyophilisée pour administration parentérale comprenant,
a) du tosylate d'omadacycline en quantité équivalente de 50 mg à 100 mg d'omadacycline/flacon; et
b) de l'arginine en quantité équivalente de 50 mg à 200 mg/flacon et du bisulfite de sodium en quantité équivalente de 0,5 mg à 2 mg/flacon comme agents stabilisants;
c) de la solution d'hydroxyde de sodium diluée q.s. et/ou
d) de la solution d'acide chlorhydrique diluée q.s. pour réguler le pH;
le pH de la solution, avant lyophilisation, est compris entre 5,6 et 6,6.

2. Composition pharmaceutique stable lyophilisée selon la revendication 1, dans laquelle du tosylate d'omadacycline équivalent à de l'omadacycline et à de l'arginine est dans un rapport de 1:1 à 1:2 et du tosylate d'omadacycline équivalent à de l'omadacycline et à du bisulfite de sodium est dans un rapport de 1:0,005 à 1:0,02.

3. Composition pharmaceutique stable lyophilisée selon les revendications 1 et 2, comprenant;
a) du tosylate d'omadacycline dans une quantité de 100 mg d'omadacycline/flacon;
b) de l'arginine en quantité de 100 mg/flacon et du bisulfite de sodium en quantité de 1 mg/flacon en tant qu'agents stabilisants;
c) de la solution d'hydroxyde de sodium diluée q.s. et/ou
d) de la solution d'acide chlorhydrique diluée q.s. pour réguler le pH;
le pH de la solution, avant lyophilisation, est compris entre 5,6 et 6,6.

4. Composition pharmaceutique stable lyophilisée selon l'une des revendications 1 à 3, dans laquelle le procédé d'augmentation de la stabilité et de réduction des impuretés de la composition pharmaceutique lyophilisée de tosylate d'omadacycline dans une solution aqueuse comprend une étape de combinaison du tosylate d'omadacycline avec de l'arginine et du bisulfite de sodium comme agents stabilisants en milieu aqueux et le maintien de la solution principale dans une plage de pH de 5.6 à 6.6.

5. Composition pharmaceutique stable lyophilisée selon la revendication 4, dans laquelle la forme lyophilisée a moins de 5% d'impuretés même après 6 mois à 40°C et 75% HR.
